(19) Europäisches Patentamt
European Patent Office
Office européen des brevets

(11) **EP 3 068 456 B1**

(12) **EUROPÄISCHE PATENTSCHRIFT**

(45) Veröffentlichungstag und Bekanntmachung des Hinweises auf die Patenterteilung:
**28.08.2019   Patentblatt 2019/35**

(21) Anmeldenummer: **14798796.0**

(22) Anmeldetag: **12.11.2014**

(51) Int Cl.:
*A61M 1/00* *(2006.01)*      *A61M 27/00* *(2006.01)*
*A61M 3/02* *(2006.01)*      *B01D 63/02* *(2006.01)*
*A61F 13/00* *(2006.01)*      *B01D 69/00* *(2006.01)*

(86) Internationale Anmeldenummer:
**PCT/EP2014/074312**

(87) Internationale Veröffentlichungsnummer:
**WO 2015/071280 (21.05.2015 Gazette 2015/20)**

(54) **WUNDVERSORGUNGSSYSTEM MIT EINER MATTE AUS KAPILLARMEMBRANEN**

WOUND CARE SYSTEM WITH A MAT MADE OF CAPILLARY MEMBRANES

SYSTÈME DE TRAITEMENT DES PLAIES DOTÉ D'UN MATELAS CONSTITUÉ DE MEMBRANES CAPILLAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2013   EP 13192648**

(43) Veröffentlichungstag der Anmeldung:
**21.09.2016   Patentblatt 2016/38**

(73) Patentinhaber: **3M Innovative Properties Company**
**St. Paul, MN 55133-3427 (US)**

(72) Erfinder:
• **MEIXNER, Carsten**
  **42499 Hückeswagen (DE)**
• **KORBI, Haythem**
  **42289 Wuppertal (DE)**

(74) Vertreter: **Hettstedt, Stephan et al**
**3M Deutschland GmbH**
**3M Office of Intellectual Property Counsel**
**Carl-Schurz Str. 1**
**41453 Neuss (DE)**

(56) Entgegenhaltungen:
EP-A2- 0 442 147      WO-A1-2010/037092
DE-A1- 4 308 850      DE-B3-102006 042 732

EP 3 068 456 B1

## Beschreibung

Die Erfindung betrifft ein Wundversorgungssystem zum Einbringen in eine Wunde bzw. zum Aufbringen auf eine Hautwunde und unter einen Wundverband, umfassend eine einlagige Anordnung aus parallel zueinander angeordneten Kapillarmembranen mit einer porösen, semipermeablen Wand und einem Lumen und mindestens einem offenen Ende, wobei die Kapillarmembranen einen Außendurchmesser im Bereich von 50 bis 5000 $\mu$m und eine Wandstärke im Bereich von 5 bis 1000 $\mu$m aufweisen und wobei die Kapillarmembranen mit ihrem mindestens einen offenen Ende mit mindestens einer gemeinsamen Versorgungsleitung mit einer Wand und einem Lumen in Fluidverbindung stehen, so dass durch die Versorgungsleitung und die Kapillarmembranen Flüssigkeiten, Medien, Gase und/oder andere Stoffe durchleitbar sind.

Die moderne Wundversorgung verfolgt das Ziel, bei der Wundversorgung ein feuchtes Wundmileu zu schaffen, das bei der Heilung ablaufende Prozesse fördert. Je nach Heilungsphase müssen moderne, aktive Wundauflagen deshalb in der Lage sein, große Mengen an Exsudat abzuführen oder die Wunde feucht zu halten. In der Wundbehandlung kommt es darauf an, für einen verbesserten Flüssigkeits-/Stoffaustausch, für das Einbringen von Faktoren/Medikamenten und/oder für eine verbesserte Flüssigkeits-/Sekrets- und/oder Stoffentfernung in der Wunde bzw. in die Wunde zu sorgen. Anwendungen schließen die Nutzung solcher Wundauflagesysteme in einer Weichteilwunde, in einer abdominellen Wunde und auf einer Hautwunde ein.

Ein Verfahren und eine Vorrichtung zur Entfernung von Sekret bzw. Exsudat aus Wunden ist kommerziell bekannt als V.A.C.® Therapy System (Fa. KCI, USA). Bei diesem System wird für ein alternierendes Einbringen von Flüssigkeit in die Wunde und ein nachfolgendes also auch alternierendes und damit nicht kontinuierliches Ausführen von Flüssigkeit aus der Wunde gesorgt. Ein in die Wunde eingebrachtes Schaumstoffmaterial, das bei Unterdruck Kräfte auf die Wunde ausübt, soll bei diesem System die Wundheilung begünstigen.

In der DE 10 2006 042 732 wird ein Kapillarmembransystem zur Wundbehandlung beschrieben, bei dem die Wunde über eine Hohlfasermembrananordnung aus bis zu 1000 Hohlfasern mit mindestens einer gemeinsamen Zu- und mindestens einer gemeinsamen Ableitung im Sinne der Durchströmung eines Kapillarbettes perfundiert und versorgt werden und eine Antibiotika- sowie Wachstumsfaktorenperfusion ermöglicht werden soll. Dabei soll eine gleichmäßige Stoffverteilung unter kontinuierlicher Perfusion auch unter Erzeugung eines moderaten Unterdruckes ermöglicht werden. Die DE 10 2006 042 732 führt aus, dass für eine optimale Ver- und Entsorgung weitere Kapillarmembransysteme von Vorteil sind.

Wenngleich sich bereits mit den in der DE 10 2006 042 732 beschriebenen Kapillarmembransystemen Fortschritte bei der Wundbehandlung erzielen lassen, besteht Bedarf an einfachen und effizienten Wundversorgungssystemen, mit denen zum einen die bei der Wundbehandlung notwendigen Vorgänge, wie Spülen und Desinfizieren, ohne Entfernen des Verbandes durchgeführt werden können, die erforderlichenfalls eine Ernährung, Elektrolytaustausch und/oder Detoxifikation oder auch eine Wachstumsfaktorenversorgung oder eine Versorgung mit Antibiotika ermöglichen und die eine einfache und sichere Handhabung erlauben.

Es ist Aufgabe der vorliegenden Erfindung, ein derartiges Wundversorgungssystem zur Verfügung zu stellen.

Die Aufgabe wird durch ein Wundversorgungssystem gelöst, welches eine einlagige Anordnung aus parallel zueinander angeordneten Kapillarmembranen mit einer porösen, semipermeablen Wand und einem Lumen und mindestens einem offenen Ende umfasst,

- wobei die Kapillarmembranen einen Außendurchmesser im Bereich von 50 bis 5000 $\mu$m und eine Wandstärke im Bereich von 5 bis 1000 $\mu$m aufweisen,
- wobei die Kapillarmembranen mit ihrem mindestens einen offenen Ende mit mindestens einer gemeinsamen Versorgungsleitung mit einer Wand und einem Lumen verbunden sind, so dass durch die Versorgungsleitung und die Kapillarmembranen Flüssigkeiten, Medien, Gase und/oder andere Stoffe durchleitbar sind, und
- wobei die Versorgungsleitung mit einer Einheit zur Versorgung bzw. Entsorgung verbindbar ist,

dadurch gekennzeichnet,

- dass die Kapillarmembranen mit ihrem mindestens einen offenen Enden so an ihrem äußeren Umfang fluiddicht in der Wand der mindestens einen gemeinsamen Versorgungsleitung eingebettet sind, dass zwischen dem Lumen der Versorgungsleitung und dem Lumen der Kapillarmembranen eine Fluidverbindung besteht,
- dass die Anordnung aus parallel zueinander angeordneten Kapillarmembranen des Weiteren mehrere zueinander beabstandete und zueinander parallel verlaufende Verbindungselemente aufweist, mittels derer die Kapillarmembranen miteinander zu einer Matte verbunden und durch die Verbindungselemente zueinander auf Abstand gehalten sind,
- dass der Abstand der Kapillarmembranen zueinander in der Matte das 1 bis 10 fache des Außendurchmessers der Kapillarmembranen beträgt, wobei der Abstand von den Längsachsen der Kapillarmembranen gemessen wird und

2

- dass der Abstand der Verbindungselemente zueinander im Bereich von 1 bis 50 mm liegt.

Es hat sich gezeigt, dass mit einem solchen Wundversorgungssystem eine nahezu homogene Versorgung einer Wunde z.B. mit Nährlösung oder auch eine nahezu homogene Entsorgung z.B. von Exsudat aus einer Wunde ermöglicht wird, wobei die Konfiguration des Wundversorgungssystems gleichzeitig eine sichere und einfache Handhabung erlaubt. In der Anwendung kann dazu das Wundversorgungssystem in die Wunde eingelegt und z.B. mittels einer semiokklusiven transparenten Folie abgedeckt werden, um die Wunde vor dem Austrocknen oder vor Infektionen zu schützen. Die mindestens eine Versorgungsleitung wird dann unter der Folie aus dem Wundbereich herausgeführt und z.B. über eine Pumpe mit einer Versorgungseinheit für z.B. eine Nährlösung, z.B. einem Vorratsbehälter für die Nährlösung verbunden. Die Anordnung der Kapillarmembranen kann auch zur Absaugung von Exsudat aus der Wunde eingesetzt werden. In diesem Fall ist die mindestens eine Versorgungsleitung mit einer Einheit zur Entsorgung, insbesondere einer Unterdruckeinheit verbunden.

Die Versorgung der Wunde mit einer Flüssigkeit und die Absaugung von Exsudat aus der Wunde kann über die Anordnung der Kapillarmembranen auch intermittierend durchgeführt werden, indem die mindestens eine Versorgungsleitung über beispielsweise ein T-Stück über eine erste Teilleitung mit einer Flüssigkeitsvorlage und über eine zweite Teilleitung mit einer Unterdruckeinheit verbunden ist. Mittels ansteuerbarer Absperrventile kann über vorgegebene Zeitintervalle entweder eine Versorgung mit Flüssigkeit oder eine Absaugung von Exsudat erfolgen.

In einer Ausführungsform kann das Wundversorgungssystem des Weiteren eine taschenförmige Wundauflage umfassen, wobei die taschenförmige Wundauflage an ihrem äußeren Rand geschlossen ist und eine Oberseite, eine Unterseite und ein Tascheninneres aufweist, wobei die Unterseite und die Oberseite jeweils aus einem flächigen Material ausgebildet sind und die Unterseite permeabel für Fluide ist, wobei die Anordnung aus parallel zueinander angeordneten Kapillarmembranen im Tascheninneren angeordnet ist und wobei die mindestens eine Versorgungsleitung außerhalb der taschenförmigen Wundauflage mit einer Einheit zur Versorgung bzw. Entsorgung verbindbar ist.

Die taschenförmige Wundauflage mit dem Wundversorgungssystem kann so in eine zu behandelnde Wunde eingelegt werden, dass die Unterseite mit der Wunde in Kontakt ist. Über das Wundversorgungssystem kann der Wunde beispielsweise Flüssigkeit z.B. in Form einer Nährlösung zugeführt werden, die sich nach Austritt aus den Kapillarmembranen in der Tasche verteilt und über die semipermeable Taschenunterseite an die Wunde abgegeben wird. Über die mindestens eine Versorgungsleitung kann dann das Wundversorgungssystem z.B. über eine Pumpe mit einer Versorgungseinheit für die Flüssigkeit, z.B. einem Vorratsbehälter für die Nährlösung verbunden werden.

Die taschenförmige Wundauflage kann vorzugsweise so ausgeführt sein, dass sich die Verbindung der Anordnung aus parallel zueinander angeordneten Kapillarmembranen bzw. der Kapillarmembranen mit der mindestens einen Versorgungsleitung im Tascheninneren befindet und die mindestens eine Versorgungsleitung über eine an ihren äußeren Querschnitt fluiddicht angepasste Durchtrittsöffnung aus der taschenförmigen Wundauflage herausführt. Ebenso kann die Verbindung der Anordnung aus parallel zueinander angeordneten Kapillarmembranen bzw. der Kapillarmembranen mit der mindestens einen Versorgungsleitung außerhalb der taschenförmigen Wundauflage auf der Oberseite angeordnet sein und die Anordnung aus parallel zueinander angeordneten Kapillarmembranen zur Verbindung mit der mindestens einen Versorgungsleitung über eine fluiddicht angepasste Durchtrittsöffnung aus der taschenförmigen Wundauflage herausführen.

Wesentliche Merkmale des Wundversorgungssystems sind, dass die Kapillarmembranen durch den mattenförmigen Aufbau sich zueinander in einem gleichförmigen Abstand befinden, in dem sie durch die Verbindungselemente gehalten werden, und dass die Kapillarmembranen innerhalb der Matte in einer solchen Dichte vorliegen, dass der Abstand der Kapillarmembranen zueinander in der Matte das 1 bis 10-fache des Außendurchmessers der Kapillarmembranen beträgt, wobei der Abstand von den Längsachsen der Kapillarmembranen gemessen wird Dabei sind Matten bevorzugt, in denen der Abstand der Kapillarmembranen zueinander in der Matte das 1,05 bis 6-fache des Außendurchmessers der Kapillarmembranen beträgt. Besonders bevorzugt sind Abstände der Kapillarmembranen zueinander in der Matte im Bereich des 1,05 bis 3-fachen des Außendurchmessers der Kapillarmembranen. In einer weiteren besonders bevorzugten Ausführungsform sind dabei Abstände der Kapillarmembranen zueinander in der Matte von mehr als dem 1,5 fachen des Außendurchmessers der Kapillarmembranen. Es wurde gefunden, dass sich hiermit eine sichere Trennung der Kapillarmembranen voneinander erreichen lässt.

Gleichzeitig ist es auch im Hinblick auf eine gute homogene Versorgung der zu behandelnden Wunde wichtig, dass die Kapillarmembranen in der Anordnung der Kapillarmembranen mittels mehrerer zueinander beabstandeter und zueinander parallel verlaufender Verbindungselemente miteinander zu einer Matte verbunden und durch die Verbindungselemente zueinander auf Abstand gehalten sind. Die Verbindungselemente befinden sich zueinander in einem definierten Abstand, der vorzugsweise im Bereich von 1 bis 50 mm liegt, wobei ein Abstand im Bereich von 3 bis 20 mm besonders bevorzugt und ein solcher im Bereich von 4 bis 6 mm bestens geeignet ist. Es hat sich nämlich gezeigt, dass die Kontaktstellen zwischen den Kapillarmembranen und den Verbindungselementen z.B. bei einer Versorgung der zu behandelnden Wunde z.B. mit einer Nährlösung in erheblichem Maße Verteilung der Flüssigkeit über der Fläche der Anordnung der Kapillarmembranen fördern. So wurde beobachtet, dass es an den Kontaktstellen zu einer Begünstigung des Austritts von Flüssigkeit aus den Kapillarmembranen kommt.

Dabei weisen die Kapillarmembranen vorzugsweise einen Außendurchmesser im Bereich von 200 bis 1500 μm auf. Ebenso sind Kapillarmembranen mit einer Wandstärke im Bereich von 20 bis 400 μm, wobei deren Außendurchmesser vorzugsweise in den zuvor genannten Bereichen liegt.

In einer Ausgestaltung des Wundversorgungssystems ist die Anordnung der Kapillarmembranen für die Zuführung bzw. Abführung von flüssigen Medien ausgelegt. Um dann eine gleichmäßige Ver- bzw. Entsorgung der Wunde zu gewährleisten, weisen in einer bevorzugten Ausführungsform die Kapillarmembranen eine hohe Permeabilität für Flüssigkeiten auf. Vorzugsweise liegt hierbei der Transmembranfluss für Wasser der Kapillarmembranen im Bereich von 0,01 bis 50 ml/(min·cm$^2$·bar).

Die Anzahl der Kapillarmembranen in der Anordnung der Kapillarmembranen des Wundversorgungssystems hängt natürlich in erster Linie von der Größe des Wundversorgungssystems und damit von der Größe der Kapillarmembrananordnung ab, die wiederum auf die Größe der damit zu behandelnden Wunde abzustimmen ist. Die Anordnung aus Kapillarmembranen kann daher aus etwa 10 bis zu mehreren hundert oder tausend zueinander parallel angeordneten Kapillarmembranen aufgebaut sein.

Die zueinander parallel angeordneten Kapillarmembranen sind an mindestens einem ihrer Enden so an ihrem äußeren Umfang fluiddicht in der Wand der mindestens einen Versorgungsleitung eingebettet, dass zwischen dem Lumen der Versorgungsleitung und dem Lumen der Kapillarmembranen eine Fluidverbindung besteht und durch die Versorgungsleitung und die Kapillarmembranen Flüssigkeiten, Medien, Gase und/oder andere Stoffe durchleitbar sind. Vorzugsweise ist die mindestens eine Versorgungsleitung an ihrem einen Ende offen und mit einer Versorgungseinheit oder einer Einheit zur Entsorgung verbindbar, während das andere Ende der mindestens einen Versorgungsleitung geschlossen ist. Die Einbettung kann mittels üblicher Kleber wie z.B. härtbarer Silikonmaterialien, Polyurethanharzen oder einem Epoxidharzen erfolgen. Bevorzugt werden wegen ihrer besseren Flexibilität härtbare Silikonmaterialien eingesetzt. Im Falle, dass die Kapillarmembranen nur mit einem ihrer Enden in einer Versorgungsleitung eingebettet sind, ist das andere, gegenüberliegende Ende der Kapillarmembranen geschlossen, beispielsweise durch Verschweißen oder Verkleben. Die Kapillarmembranen können auch an ihren beiden Enden offen und mit diesen beiden Enden auf einer Seite der Anordnung in eine Versorgungsleitung eingebettet sein, wobei die Kapillarmembranen dann an ihrem freien Ende U-förmig ausgebildet sind und dadurch dort geschlossen sind. In diesen Fällen werden die Kapillarmembranen im Dead-end Modus betrieben.

Insbesondere bei breiteren Wundversorgungssystemen ist eine Ausführungsform der einlagigen Kapillarmembrananordnung von Vorteil, bei dem die parallel zueinander angeordneten Kapillarmembranen an ihren beiden Enden offen und die entgegengesetzten Enden in jeweils eine Versorgungsleitung eingebettet sind, wobei sich die Versorgungsleitungen sich dann bevorzugt an gegenüberliegenden Seiten der Kapillarmembrananordnung befinden. Auch in diesem Fall ist die Einbettung so ausgeführt, dass die Kapillarmembranen an ihrem äußeren Umfang fluiddicht eingebettet sind und zwischen dem Lumen der jeweiligen Versorgungsleitung und dem Lumen der Kapillarmembranen eine Fluidverbindung besteht. Eine derartige Ausführungsform mit zwei Versorgungsleitungen erlaubt eine Versorgung und/oder Entsorgung über die Kapillarmembrananordnung im cross-flow Modus. Es kann jedoch auch bei einer Beaufschlagung der Kapillarmembrananordnung im dead-end Modus insbesondere bei breiteren Kapillarmembrananordnungen für eine gute Homogenität der Versorgung bzw. Entsorgung über der Fläche der Wunde vorteilhaft sein, wenn die Kapillarmembrananordnung an beiden Enden der Kapillarmembranen eine Versorgungsleitung aufweist.

Der Durchmesser der mindestens einen Versorgungsleitung richtet sich in erster Linie nach dem Außendurchmesser der in sie eingebetteten Kapillarmembranen. Daher weist die mindestens eine Versorgungsleitung bevorzugt einen Innendurchmesser im Bereich von 0,1 bis 10 mm auf. Ebenso ist bevorzugt, wenn die Wandstärke des flexiblen Silikonschlauchs im Bereich von 0,1 bis 5 mm liegt. Im Falle der Verwendung einer Versorgungsleitung mit nichtkreisförmigem Querschnitt wird als Innendurchmesser der äquivalente Durchmesser d = 4A/U des Innenquerschnitts angesetzt mit A als der Fläche des Innenquerschnitts und U als dessen Umfang. Beispielsweise kann die Versorgungsleitung auch einen ovalen, oder näherungsweise quadratischen oder rechteckigen Innenquerschnitt aufweisen. Für die mindestens eine Versorgungsleitung hat sich beispielsweise ein Silikonschlauch als geeignet erwiesen, durch dessen Wand die Kapillarmembranenden hindurchtreten und in der sie eingeklebt sind. Vorzugsweise ist die mindestens eine gemeinsame Versorgungsleitung ein flexibler Silikonschlauch. Die Einbettung bzw. das Einkleben in die Wand der Versorgungsleitung kann mittels üblicher Kleber wie z.B. mittels härtbarer Silikonmaterialien, Polyurethanharzen oder einem Epoxidharzen erfolgen.

Erfindungsgemäß sind die zueinander parallel angeordneten Kapillarmembranen mittels mehrerer zueinander beabstandeter und zueinander parallel verlaufender Verbindungselemente miteinander verbunden und durch die Verbindungselemente zueinander auf Abstand gehalten, wobei die Verbindungselemente die Kapillarmembranen an ihrem äußeren Umfang berühren oder die Kapillarmembranen umschlingen. Die Verbindungselemente weisen entlang ihrer Längserstreckung keine geschlossenen Strömungskanäle auf, sind also folglich entlang ihrer Längserstreckung nicht von Fluiden durchströmbar. Die Verbindungselemente können quer zu den parallel zueinander angeordneten Kapillarmembranen verlaufen oder auch unter einem anderen Winkel. Bei den Verbindungselementen kann es sich um Klebestreifen handeln oder beispielsweise auch um strangförmige Elemente aus einem Silikonmaterial. In einer bevorzugten Ausführungsform sind die Kapillarmembranen mittels garnförmiger Verbindungselemente zu einer Matte verbunden. Besonders bevorzugt handelt es sich bei den Verbindungselementen um textile Multifilamentgarne. Besonders bewährt haben sich als textile Multifilamentgarne multifile Polyestergarne, Polypropylengarne oder Polytetrafluorethylengarne. Bestens geeignet hydrophile Garne, vorzugsweise aus Polyester. Wie erläutert, hat sich gezeigt, dass solchermaßen ausgeführte Verbindungselemente unterstützend bei der Verteilung von in die Wunde zugeführten Flüssigkeiten wirken.

Bei der Kapillarmembranmatte kann es sich in einer bevorzugten Ausführungsform um eine Wirkmatte handeln, in der die Kapillarmembranen und die Verbindungsfäden miteinander verwirkt sind und bei der die Kapillarmembranen quer zur Erstreckungsrichtung der Kapillarmembranmatte verlaufen und die Länge der Kapillarmembranen durch die Mattenbreite bestimmt ist. In einer weiteren bevorzugten Ausführungsform kann es sich bei der Kapillarmembranmatte um eine Webmatte handeln, in der die Kapillarmembranen und die Verbindungsfäden miteinander verwoben sind und bei der die Kapillarmembranen in Erstreckungsrichtung oder Laufrichtung der Kapillarmembranmatte verlaufen und die textilen Fäden quer dazu. Kapillarmembranwirkmatten und -webmatten sowie Möglichkeiten ihrer Herstellung werden beispielsweise in der DE 38 39 567, der DE 43 08 850 und in der EP 0 442 147 beschrieben. Insbesondere mittels der Wirktechnologie lassen sich auf einfache Weise Matten herstellen, bei denen die Kapillarmembranen an ihrem freien Ende U-förmig ausgebildet und dort verschlossen sind. Derartige Matten können durch mäanderförmige Ablage einer Kapillarmembran in zueinander parallele Stränge, die durch die Wirkfäden miteinander verbunden sind, hergestellt werden. Dabei werden nach Fertigstellung der Wirkmatte die U-förmig ausgebildeten Enden an mindestens einer Seite der Wirkmatte abgetrennt und die dabei entstehenden offenen Enden der Kapillarmembranen dann in der mindestens einen Versorgungsleitung eingebettet. Im Falle, dass die U-förmig ausgebildeten Enden an beiden Seite der Wirkmatte abgetrennt werden, werden die entstehenden gegenüberliegenden offenen Enden jeweils in Versorgungsleitungen eingebettet.

Die Form der einlagigen Anordnung aus parallel zueinander angeordneten Kapillarmembranen in ihrer flächigen Erstreckung kann beliebig sein, soweit dies für Anordnungen aus zueinander parallelen Kapillarmembranen möglich ist. In der einfachsten Ausführung weist die Anordnung aus zueinander parallelen Kapillarmembranen eine quadratische oder rechteckige Form auf. Es ist jedoch z.B. bei Anordnungen, bei denen die Kapillarmembranen nur an einem ihrer Enden in eine Versorgungsleitung eingebettet sind, möglich, dass beispielsweise durch entsprechend angepasstes Abschweißen der freien, verschlossenen Enden der zueinander parallelen Kapillarmembranen eine bogenförmige Kontur ausgebildet wird. Ebenso ist es möglich, dass die Anordnung aus zueinander parallelen Kapillarmembranen z.B. auch eine trapezförmige Kontur aufweist.

Als Materialien für die Kapillarmembranen kommen grundsätzlich alle im Stand der Technik bekannten organischen Polymere in Frage, die zur Ausbildung von Kapillarmembranen geeignet sind, wobei diese Polymere eine gute Biokompatibilität ausweisen müssen. Darüber hinaus ist es auch erforderlich, dass das Membranpolymer eine Sterilisation des Wundversorgungssystems beispielsweise über Dampfsterilisation, Sterilisation mittels $\gamma$-Strahlung oder Sterilisation mittels Ethylenoxid erlaubt. Dabei können die organischen Polymere natürliche Polymere sein oder Polymere, die auf synthetischen Wege hergestellt wurden. Natürliche Polymere sind insbesondere solche auf Basis von zellulosischen Polymeren, was Polymere, die sog. polymeranalogen Reaktionen unterzogen worden sind, ebenfalls umfasst. Beispiele für Polymere auf Basis von Zellulose sind solche aus regenerierter Zellulose, Zelluloseazetat oder modifizierter Zellulose wie z.B. Zelluloseester, Zelluloseäther, mit Benzylgruppen modifizierte Zellulose (Benzylzellulose) oder mit Diethylaminoethyl modifizierte Zellulose oder Mischungen dieser zellulosischen Polymere. Des Weiteren können auch Polymere auf der Basis von Chitin, bzw. Chitosan zum Einsatz kommen.

Als auf synthetischem Wege hergestellte Polymere, d.h. als synthetische Polymere können solche verwendet werden, die aus Polyolefinen, Polyamiden, Polyacrylnitrilen, Polycarbonaten, Polyestern oder Sulfonpolymeren sowie daraus gewonnen Modifikationen, Blends, Mischungen oder Copolymere dieser Polymere bestehen. Vorzugsweise werden solche verwendet, die auf Sulfonpolymeren, wie insbesondere Polysulfon oder Polyethersulfon, basieren. Den synthetischen Polymeren können weitere Polymere wie z.B. Polyethylenoxid, Polyhydroxyether, Polyethylenglykol, Polyvinylalkohol oder Polycaprolacton als Zusatzstoffe beigemischt werden. Die Kapillarmembranen können darüber hinaus noch eine Beschichtung mit einem Additiv aufweisen. Bevorzugt enthalten solche Kapillarmembranen ein Hydrophilierungsmittel, z.B. Polyvinylpyrrolidon oder auch hydrophile Modifikationen dieser Polymere.

Die Kapillarmembranen können mit Blick auf bestimmte Anwendungen z.B. über Ankopplung funktioneller Gruppen modifiziert sein oder beispielsweise mit Heparin oder einem Antibiotikum oder mehreren Antibiotika beschichtet sein.

Neben der einlagigen Anordnung aus parallel zueinander angeordneten Kapillarmembranen kann das Wundversorgungssystem weitere Komponenten aufweisen wie z.B. mindestens eine weitere Anordnung von Kapillarmembranen, bei der die Kapillarmembranen der weiteren Anordnung Membranen zur Oxygenation sind, d.h. Membranen, über die eine Zuführung von Sauerstoff zur Wunde möglich ist. Derartige Membranen werden beispielweise in der in der EP-A-1 144 096, der EP-A-0 299 381 oder der DE-A-28 33 493 offenbart. Auch die Kombination mit einer weiteren mattenförmigen Anordnung von semipermeablen Kapillarmembranen ist möglich, so dass z.B. über die erste Anordnung von Kapillarmembranen eine Versorgung der Wunde mit Wachstumsfaktoren oder mit Antibiotika und über die weitere Anordnung eine Temperierung oder eine pH-Wert Regulierung erfolgen kann. Dabei können die jeweiligen mattenförmigen Anordnungen aufeinander gelegt werden. Es ist jedoch auch möglich, dass zwei unterschiedliche Kapillarmembranen miteinander zu einer Matte verbunden sind, wobei die unterschiedlichen Kapillarmembranen mit ihren Enden in unterschiedliche Versorgungsleitungen eingebettet sind, die vorzugsweise an entgegengesetzten Seiten der Matte angeordnet sind. Solche Matten können beispielsweise durch Verwirken von zueinander versetzt angeordneten, mäanderförmig abgelegten Kapillarmembranen erhalten werden, bei denen die U-förmigen Umlenkungen der Kapillarmembranen sich an unterschiedlichen Positionen über der Mattenbreite befinden. Durch Schneiden der jeweils außenliegenden U-förmigen Umlenkungen werden die Kapillarmembranen an jeweils nur einer Seite der Matte geöffnet und können dort in eine Versorgungsleitung eingebettet werden.

Des weiteren kann das Wundversorgungssystem zusätzlich zu der einlagigen Anordnung aus parallel zueinander angeordneten Kapillarmembranen beispielsweise ein Drainagesystem aufweisen, mittels dessen eine separate Abführung von Exsudat möglich ist. In einer Ausführungsform kann das Wundversorgungssystem einen Saugschwamm aufweisen, welcher über eine Absaugleitung für Exsudat verfügt. Das Drainagesystem kann auch als eine weitere Kapillarmembranmatte ausgebildet sein, es kann jedoch auch in Gestalt eines Drainagekatheters vorliegen, bei dem es sich um ein Schlauchstück, beispielsweise aus einem Silikonmaterial, oder um ein Röhrchen handeln kann. Ein solcher Drainagekatheter kann in seiner Wand Perforierungen aufweisen, über die nach Anschluss des Drainagekatheders an eine Unterdruckeinheit z.B. Exsudat aus der Wunde bzw. aus dem Inneren der taschenförmigen Wundauflage und aus der Wunde abgesaugt werden kann.

Im Falle, dass das Wundversorgungssystem des Weiteren eine taschenförmigen Wundauflage umfasst, in der die Anordnung aus parallel zueinander angeordneten Kapillarmembranen angeordnet ist, erstreckt sich die mindestens eine Anordnung aus parallel zueinander angeordneten Kapillarmembranen flächig im Tascheninneren. Die Dimensionen der Anordnung aus parallel zueinander angeordneten Kapillarmembranen ergeben sich aus deren äußeren Abmessungen in der flächigen Erstreckung. Vorzugsweise füllt Anordnung der Kapillarmembranen hinsichtlich ihrer flächigen Erstreckung das Tascheninnere der taschenförmigen Wundauflage in dessen flächiger Erstreckung mindestens zu 20 % und besonders bevorzugt mindestens zu 50 % aus. Von besonderem Vorteil ist es, wenn die Anordnung der Kapillarmembranen hinsichtlich ihrer flächigen Erstreckung das Tascheninnere der taschenförmigen Wundauflage in dessen flächiger Erstreckung mindestens zu 70 % ausfüllt, wobei auch Füllgrade im Bereich von 90 % realisiert werden können. Dabei ist es von Vorteil, wenn die Anordnung der Kapillarmembranen in der taschenförmigen Wundauflage mittig angeordnet ist.

Die taschenförmige Wundauflage kann beliebige Konturen aufweisen. Vorzugsweise ist die Kontur jedoch rund, oval, quadratisch oder rechteckig. Unterseite und Oberseite der taschenförmigen Wundauflage sind am äußeren Rand bzw. an den äußeren Kanten der Wundauflage beispielsweise durch Verschweißen oder Verkleben miteinander verbunden. Zur Verklebung sind u.a. Silikonstreifen geeignet, die ausgehärtet werden. Bei rechteckigen oder bei quadratischen taschenförmigen Wundauflagen weist das darin angeordnete flächenförmig ausgebildete Kapillarmembransystem vorzugsweise ebenfalls eine rechteckige oder quadratische Kontur auf. Bei runden oder ovalen taschenförmigen Wundauflagen ist das darin befindliche mindestens eine flächenförmig ausgebildete Kapillarmembransystem zweckmäßigerweise ebenfalls quadratisch oder rechteckig ausgebildet, wobei hinsichtlich der Abmessungen ebenfalls die zuvor genannten Dimensionen gelten. Es kann jedoch auch an die Kontur der taschenförmigen Wundauflage angepasst sein, beispielsweise durch entsprechend angepasstes Abschweißen der nicht eingebetteten Enden der Kapillarmembranen bei einer Anordnung der Kapillarmembranen mit nur einer Versorgungsleitung, so dass sich an dieser Kante der Anordnung der Kapillarmembranen eine bogenförmige Kontur ergibt.

Die Unterseite der taschenförmigen Wundauflage ist gegenüber Fluiden durchlässig, d.h. permeabel. Dabei kann die Unterseite beispielsweise aus einem vliesförmigen, flächigen Material, einem gitterförmigen oder netzartigen Material, einer perforierten Folie oder einer semipermeablen mikroporösen Flachmembran bestehen. In einer vorteilhaften Ausführungsform besteht die Unterseite aus einem vliesförmigen, flächigen Material oder einer semipermeablen mikroporösen Flachmembran. Die Unterseite weist bevorzugt eine Permeabilität für Wasser von mindestens 0,01 ml/(min·cm$^2$·bar) und besonders bevorzugt von mindestens 10 ml/(min·cm$^2$·bar) auf. Bestens bewährt hat sich eine Unterseite mit einer Permeabilität für Wasser von mindestens 500 ml/(min·cm$^2$·bar).

Für Anwendungen des Wundauflagesystems, bei denen die Wunde über das Wundauflagesystem nicht nur mit Flüssigkeit versorgt wird, sondern auch eine Entsorgung, d.h. Abführung von Flüssigkeiten aus der Wunde und insbesondere eine Entsorgung von Exsudat erfolgen soll, ist es von Vorteil, wenn in der Unterseite Öffnungen vorhanden sind, wobei die Öffnungen vorzugsweise einen Durchmesser von mindestens 100 $\mu$m aufweisen. Dabei sind Durchmesser der Öffnungen von höchstens 10 mm bevorzugt und von höchstens 5 mm besonders bevorzugt. Im Falle, dass die Unterseite aus einer semipermeablen mikroporösen Flachmembran besteht, weist diese in einer vorteilhaften Ausführungsform zusätzlich Öffnungen z.B. in Form von Perforationen auf. Im Falle, dass die Öffnungen eine nicht-kreisförmige Kontur aufweisen, wird als Durchmesser der äquivalente Durchmesser D=4A/U der Öffnung angesetzt mit A als der Fläche der jeweiligen Öffnung und U als deren Umfang. Die Öffnungen können regelmäßig oder unregelmäßig über die Fläche der Unterseite verteilt sein, wobei eine regelmäßige, homogene Verteilung bevorzugt ist. Dabei kann der Abstand zwischen den Öffnungen im Bereich von 1 bis 20 mm liegen, gemessen von äußeren Rand der Öffnungen.

Unter- und die Oberseite der taschenförmigen Wundauflage können aus gleichen oder unterschiedlichen Materialien bestehen. Während die Unterseite jedoch stets gegenüber Flüssigkeiten durchlässig ist, ist die Oberseite bevorzugt aus einem fluidundurchlässigen, vorzugsweise folienförmigen Material ausgebildet, das an seiner bzw. seinen Seitenkanten fluiddicht mit der Unterseite verbunden ist. Es kann sich bei der Oberseite auch um eine semipermeable, mikroporöse Flachmembran handeln. In diesem Fall weist die Oberseite jedoch eine geringere Permeabilität gegenüber Fluiden auf als die Unterseite, um in der Anwendung eine Verteilung zugeführter Flüssigkeit auf der Unterseite der taschenförmigen Wundauflage und damit zur Wunde hin zu gewährleisten. Im Falle, dass es sich bei Unterseite und Oberseite um dieselbe oder die gleiche semipermeablen mikroporösen Flachmembran handelt, weist die Unterseite Perforationen auf.

Als Materialien für die Unter- bzw. die Oberseite der taschenförmigen Wundauflage kommen grundsätzlich dieselben organischen Polymere in Frage, die zuvor als Polymere für die Kapillarmembranen genannt wurden und die sich zu Flachfolien bzw. Flachmembranen verarbeiten lassen. Vorzugsweise sind Unter- und/oder Oberseite der taschenförmigen Wundauflage aus Polyolefinen, Polyamiden, Polyacrylnitril, Polycarbonaten Polyester oder Sulfonpolymeren sowie daraus gewonnen Modifikationen, Blends, Mischungen oder Copolymere dieser Polymere aufgebaut. Besonders bevorzugt umfassen Unter- und Oberseite als Material Sulfonpolymere, wobei Polysulfon oder Polyethersulfon bestens geeignet sind.

Im Falle, dass das Wundversorgungssystem eine taschenförmigen Wundauflage umfasst, kann ein Drainagesystem im Tascheninneren angeordnet sein, welches zur Entfernung von Exsudat aus zu behandelnden Wunden geeignet ist.

Das Drainagesystem kann vorzugsweise mindestens ein Drainagekatheter sein, der über eine an seinen Querschnitt fluiddicht angepasste Durchtrittsöffnung aus der taschenförmigen Wundauflage herausführt und mit einer Unterdruckeinheit verbindbar ist, um so in der Anwendung einen Unterdruck im Tascheninneren zu erzeugen. Bei dem mindestens einen Drainagekatheder kann es sich um ein Schlauchstück, beispielsweise aus einem Silikonmaterial, oder um ein Röhrchen handeln, das im Tascheninneren der Wundauflage angeordnet ist und über die Durchtrittsöffnung aus der Wundauflage herausführt. An dem sich im Inneren der taschenförmigen Wundauflage befindlichen Segment des mindestens einen Drainagekatheters weist dieser vorzugsweise in seiner Wand Perforierungen auf, über die nach Anschluss des mindestens einen Drainagekatheders an eine Unterdruckeinheit z.B. Exsudat aus der Wunde bzw. aus dem Inneren der taschenförmigen Wundauflage und aus der Wunde abgesaugt werden kann. Der mindestens eine Drainagekatheter weist bevorzugt einen Innendurchmesser im Bereich von 0,1 bis 15 mm auf und eine Wandstärke im Bereich von 0,1 bis 3 mm und erstreckt sich vorzugsweise über mindestens die gesamte Länge oder Breite des Tascheninneren. Der Drainagekatheter kann auch einen nicht-kreisförmigen Querschnitt aufweisen. In diesem Fall wird als Innendurchmesser der äquivalente Durchmesser $d_D = 4A_D/U_D$ des Innenquerschnitts angesetzt mit $A_D$ als der Fläche des Innenquerschnitts des Drainagekatheters und $U_D$ als dessen Umfang.

Für die Charakterisierung der Eigenschaften der im Wundauflagesystem eingesetzten Kapillarmembranen bzw. Flachmembranen werden die folgenden Messmethoden zu Grunde gelegt:

Transmembranfluss (Wasserpermeabilität) für Kapillarmembranen:

Aus den zu prüfenden Kapillarmembranen wird eine Prüfzelle mit definierter Kapillarmembranzahl und Länge gefertigt. Die Kapillarmembranen werden dafür beidseitig an ihren Enden in ein Polyurethanharz eingebettet. Nach dem Aushärten des Harzes werden die Einbettungen auf eine Länge von ca. 30 mm geschnitten, wobei die Lumina der Kapillarmembranen durch den Schnitt geöffnet werden. Die Kapillarlumina in den Einbettungen müssen auf Durchgängigkeit überprüft werden. Die freie Länge der Kapillarmembranen zwischen den Einbettungen beträgt üblicherweise 120 +/- 10 mm. Die Anzahl der Kapillarmembranen ist so zu bemessen, dass unter Berücksichtigung der freien Länge und des Innendurchmessers der Kapillarmembranen eine Filtrationsfläche von ca. 30 cm$^2$ in der Prüfzelle bereitgestellt wird.

Die Prüfzelle wird mit in eine Prüfapparatur eingebunden und mit auf 25°C temperiertem ultrafiltriertem und vollentsalztem Wasser bei einem definiertem Prüfdruck (ca. 0,4 bar) durchströmt. Die während einer Messzeit von 2 min erhaltene filtrierte Wassermenge, d.h. das während der Messung erzeugte Permeat wird gravimetrisch oder volumetrisch erfasst. Vor Beginn der Messung muss die Anlage luftfrei gespült werden. Zur Bestimmung des TMF werden in der Prüfapparatur der Eingangs- und Ausgangsdruck an der Prüfzelle gemessen. Die Messung wird bei 25°C durchgeführt.

Der Transmembranfluss TMF wird nach der Formel (I)

$$TMF = \frac{V_W}{\Delta t \cdot A_M \cdot \Delta p} \quad \left[\frac{ml}{cm^2 \cdot min \cdot bar}\right] \qquad (I)$$

ermittelt. Hierbei sind:

$V_W$ = durch die Membranprobe während der Messzeit hindurch geströmte Wasservolumen [ml]
$\Delta t$ = Messzeit [min]
$A_M$ = durchströmte Fläche der Membranprobe (üblicherweise 30 cm$^2$)
$\Delta p$ = eingestellter Druck während der Messung [bar]

Permeabilität für Wasser der Unterseite der taschenförmigen Wundauflage:

Aus dem zu prüfenden flächigen Material der Unterseite der taschenförmigen Wundauflage werden scheibenförmige Proben ausgestanzt und in einen geeigneten Probenhalter am Umfang fluiddicht so eingespannt, dass eine freie Messfläche von 17,35 cm$^2$ resultiert. Der Probenhalter befindet sich in einem Gehäuse, das von Wasser druckbeaufschlagt durchströmt werden kann. Die eingespannte Probe wird dann von auf 25°C temperiertem, vollentsalztem Wasser unter einem definierten Druck zwischen 0,1 und 0,2 bar durchströmt. Es wird das während einer Messzeit von 60 s durch die Probe hindurch geströmte Wasservolumen gravimetrisch oder volumetrisch ermittelt.
Die Permeabilität für Wasser TMF$_W$ wird nach der Formel (II)

$$TMF_W = \frac{V_W}{\Delta t \cdot A_M \cdot \Delta p} \quad \left[\frac{ml}{cm^2 \cdot min \cdot bar}\right] \qquad (II)$$

ermittelt. Hierbei sind:

$V_W$ = durch die Probe während der Messzeit hindurch geströmte Wasservolumen [ml]
$\Delta t$ = Messzeit [min]
$A_M$ = durchströmte Fläche der Probe (17,35 cm$^2$)
$\Delta p$ = eingestellter Druck während der Messung [bar]

Die Erfindung wird anhand der folgenden Figuren näher erläutert, wobei durch die Figuren der Umfang der Erfindung nicht eingeschränkt wird:
Es zeigen:

Fig. 1: Wundversorgungssystem mit einer einlagigen Matte aus Kapillarmembranen und Versorgungsleitungen an beiden Enden der Matte

Fig. 2: Wundversorgungssystem mit einer Versorgungsleitung an einem der Mattenenden und versiegelten Kapillarmembranenden am gegenüberliegenden Mattenende

Fig. 3: Wundversorgungssystem mit einer Versorgungsleitung an einem der Mattenenden sowie U-förmig ausgebildeten Kapillarmembranenden am gegenüberliegenden Mattenende

Fig. 4: Fotografie eines Segments eines erfindungsgemäßen Wundversorgungssystems mit einer einlagigen Anordnung aus parallel zueinander angeordneten Kapillarmembranen bei einem Versuch mit einer Simulationslösung

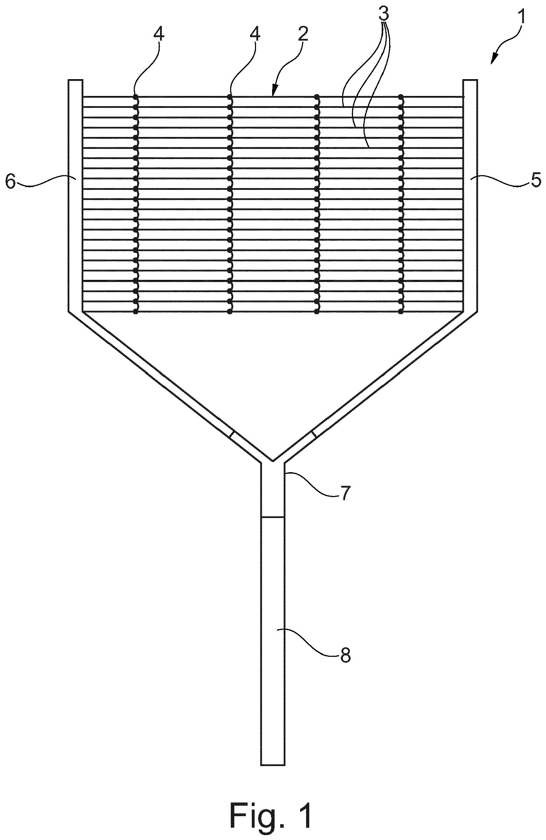

Figur 1 zeigt in der Draufsicht schematisch und nicht maßstäblich ein erfindungsgemäßes Wundversorgungssystem 1 mit einer einlagigen Anordnung 2 aus Kapillarmembranen 3. Die Kapillarmembranen 3 sind mittels zueinander parallel verlaufender Verbindungselemente 4 zu einer Matte so verbunden, dass sie zueinander parallel angeordnet und zueinander auf Abstand gehalten sind. Die Kapillarmembranen 3 sind im vorliegenden Beispiel mit ihren gegenüber liegenden Enden so in Versorgungsleitungen 5, 6 eingebettet, dass zwischen den Lumen der Versorgungsleitungen 5,6 und dem Lumen der Kapillarmembranen 3 eine Fluidverbindung besteht. Die Versorgungsleitungen 5, 6 sind über ein Y-Stück 7 zu einer gemeinsamen Leitung 8 zusammengefasst. Aus diesem Aufbau ergibt sich, dass z.B. eine Nährlösung, die über die Leitung 8 zugeführt wird, auf die Versorgungsleitungen 5, 6 aufgeteilt wird und den Kapillarmembranen 3 im dead-end Modus zugeführt wird. Die Nährlösung strömt über die porösen, semipermeablen Wände der Kapillarmembranen 3 aus diesen aus und wird über der Fläche der Anordnung 2 aus Kapillarmembranen 3 gleichmäßig der Wunde zugeführt, wobei zur gleichmäßigen Verteilung die Einhaltung der erfindungsgemäßen Bedingungen hinsichtlich einerseits des Abstandes der Kapillarmembranen 3 zueinander sowie andererseits des Abstands der Verbindungselemente zueinander einzuhalten sind.

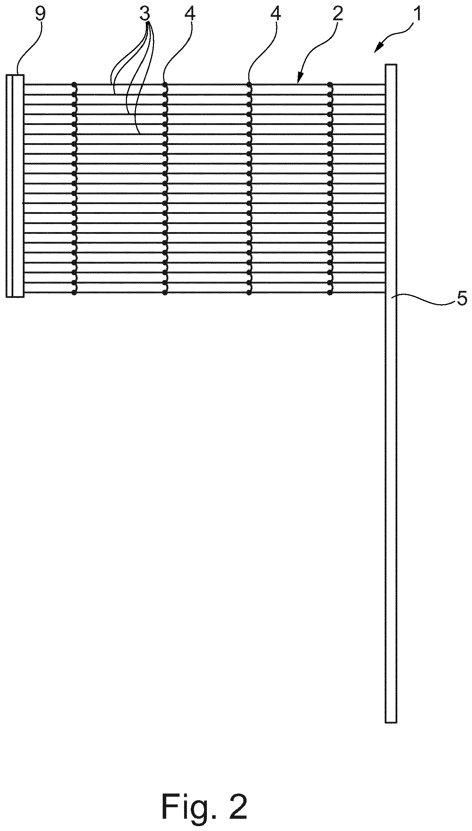

In Figur 2 ist ebenfalls schematisch und in nicht-maßstäblicher Darstellung ein Wundversorgungssystem 1 mit einer einlagigen Anordnung 2 aus Kapillarmembranen 3 gezeigt, bei dem die Kapillarmembranen 3 nur mit einem ihrer Enden in einer Versorgungsleitung 5 eingebettet sind. Die zweiten, gegenüberliegenden Enden der Kapillarmembranen 3 sind mittels z.B. eines Silikonmaterials 9 eingefasst und auf diese Weise verschlossen. Die Kapillarmembranen 3 sind ebenfalls mittels zueinander parallel verlaufender Verbindungselemente 4 zu einer Matte so verbunden, dass sie zueinander parallel angeordnet und zueinander auf Abstand gehalten sind.

Ein Wundversorgungssystem 1 wie in Figur 2 dargestellt ist in der Regel für geringere Breiten zweckmäßig, so dass trotz einer nur einseitigen Versorgung der Matte der Kapillarmembranen 3 mit z.B. einer Nährlösung eine zumindest weitgehend homogene Verteilung der Nährlösung auf die zu behandelnde Wunde über der Mattenbreite erfolgt.

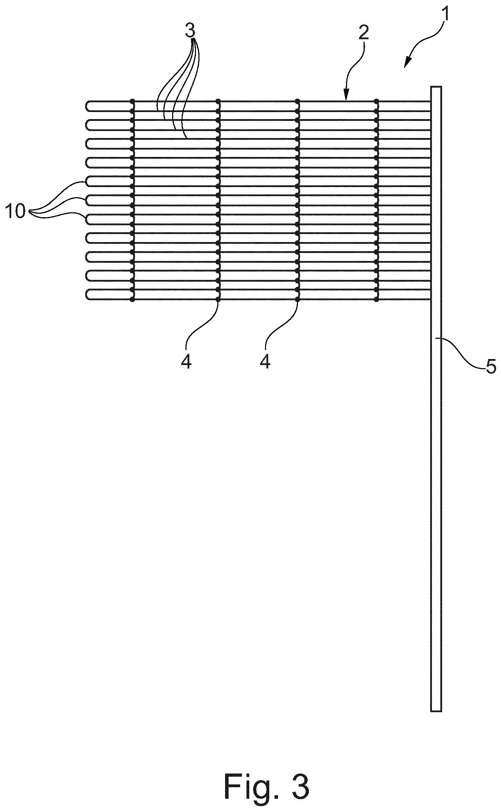

Figur 3 zeigt ebenfalls schematisch und in nicht-maßstäblicher Darstellung ein Wundversorgungssystem 1, bei dem die Kapillarmembranen 3 nur mit einer Versorgungsleitung 5 verbunden sind. Im Unterschied zu der in Figur 2 gezeigten Matte aus Kapillarmembranen sind hier die Kapillarmembranen an ihren beiden Enden offen und mit ihren beiden Enden in eine Versorgungsleitung 5 eingebettet. Die freien Enden 10 der Kapillarmembranen 3 sind an dem der Versorgungsleitung 5 gegenüberliegenden Ende der Matte U-förmig ausgebildet und dadurch dort geschlossen. Auf diese Weise erfolgt auch bei den Kapillarmembranen 3 des in Figur 3 gezeigten Wundversorgungssystems 1 die Anströmung im dead-end Modus.

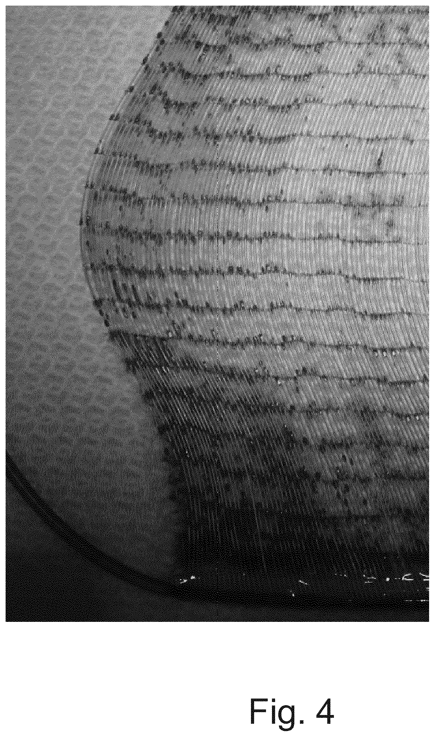

Figur 4 zeigt eine Fotographie eines Segments eines erfindungsgemäßen Wundversorgungssystems mit einer einlagigen Anordnung aus parallel zueinander angeordneten Kapillarmembranen bei einem Versuch mit einer Simulationslösung. Bei der gezeigten Segment handelt es sich um einen Teil einer Matte mit den Abmessungen 200mm in Längsrichtung (in Richtung der Kapillarmembranen) und 200mm in Querrichtung (in Richtung quer zu den Kapillarmembranen) aus Kapillarmembranen der Type MicroPES® TF10 (Membrana GmbH), die in dem gezeigten Segment in vertikaler Richtung z.T. bogenförmig geführt werden. Die Kapillarmembranen sind über Verbindungselemente in Gestalt multifiler Polyesterfäden zu einer Wirkmatte verbunden, wobei die Polyesterfäden in horizontaler Richtung verlaufen. Die Polyesterfäden haben zueinander einen Abstand von 5 mm, der Abstand der Kapillarmembranen zueinander beträgt das ca. 2,7- fache des Außendurchmessers der Kapillarmembranen (500 $\mu$m). Die Kapillarmembranen sind mit ihrem einen Ende in eine Versorgungsleitung eingebettet, die am unteren Bildrand zu erkennen ist.

Die Fotografie der Figur 4 zeigt eine Momentaufnahme zu Beginn eines Versuches zur Prüfung der Homogenität der Verteilung einer über die Kapillarmembranmatte zugeführten Flüssigkeit. Als Modellflüssigkeit wurde eine eingefärbte Safranin-Lösung eingesetzt. Die Verteilung der Flüssigkeit über die Kapillarmembranen nimmt zu dem frühen Stadium des Versuchs ihren Beginn an der Versorgungsleitung am unteren Teil der Matte. Jedoch ist deutlich zu erkennen, dass bereits zu diesem frühen Stadium eine Verteilung der Flüssigkeit auch von den Kreuzungspunkten der Kapillarmembranen mit den Polyesterfäden ausgeht, was in Figur 4 an den dunklen Punkten bzw. Linien über der Fläche der Matte zu erkennen ist. Über die Kreuzungspunkte der Kapillarmembranen mit den Verbindungselementen erfolgt also eine Begünstigung des Austritts der Flüssigkeit aus den Kapillarmembranen und damit gleichzeitig eine homogene Verteilung der Flüssigkeit über der Fläche.

## Patentansprüche

1. Wundversorgungssystem, umfassend eine einlagige Anordnung aus parallel zueinander angeordneten Kapillarmembranen (3) mit einer porösen, semipermeablen Wand und einem Lumen und mindestens einem offenen Ende,

   - wobei die Kapillarmembranen einen Außendurchmesser im Bereich von 50 bis 500 $\mu$m und eine Wandstärke im Bereich von 5 bis 1000 $\mu$m aufweisen,
   - wobei die Kapillarmembranen mit ihrem mindestens einen offenen Ende mit mindestens einer gemeinsamen Versorgungsleitung mit einer Wand und einem Lumen verbunden sind,

   **dadurch gekennzeichnet,**

- **dass** die Kapillarmembranen mit ihrem mindestens einen offenen Enden so an ihrem äußeren Umfang fluiddicht in der Wand der mindestens einen gemeinsamen Versorgungsleitung (5, 6) eingebettet sind, dass zwischen dem Lumen der Versorgungsleitung und dem Lumen der Kapillarmembranen eine Fluidverbindung besteht,
- **dass** die Anordnung aus parallel zueinander angeordneten Kapillarmembranen des Weiteren mehrere zueinander beabstandete und zueinander parallel verlaufende Verbindungselemente (4) aufweist, mittels derer die Kapillarmembranen miteinander zu einer Matte verbunden und durch die Verbindungselemente zueinander auf Abstand gehalten sind,
- **dass** der Abstand der Kapillarmembranen zueinander in der Matte das 1 bis 10 fache des Außendurchmessers der Kapillarmembranen beträgt, wobei der Abstand von den Längsachsen der Kapillarmembranen gemessen wird, und
- **dass** der Abstand der Verbindungselemente zueinander im Bereich von 1 bis 50 mm liegt.

2. Wundversorgungssystem nach Anspruch 1, **dadurch gekennzeichnet, dass** die Kapillarmembrananordnung mit zwei Versorgungsleitungen verbunden ist, wobei die Kapillarmembranen mit ihren gegenüberliegenden Enden in jeweils eine Versorgungsleitung eingebettet sind.

3. Wundversorgungssystem nach Anspruch 1 oder 2, **dadurch gekennzeichnet, dass** die Kapillarmembranen einen Transmembranfluss für Wasser im Bereich von 0,01 bis 50 ml/(min·cm$^2$·bar) aufweisen.

4. Wundversorgungssystem nach einem oder mehreren der Ansprüche 1 bis 3, **dadurch gekennzeichnet, dass** die Kapillarmembranen mittels garnförmiger Verbindungselemente zu einer Matte verbunden sind.

5. Wundversorgungssystem nach Anspruch 4, **dadurch gekennzeichnet, dass** die garnförmiger Verbindungselemente textile Multifilamentgarne sind.

6. Wundversorgungssystem nach Anspruch 5, **dadurch gekennzeichnet, dass** die textilen Multifilamentgarne multifile Polyestergarne, Polypropylengarne oder Polytetrafluorethylengarne sind.

7. Wundversorgungssystem nach einem oder mehreren der Ansprüche 4 bis 6, **dadurch gekennzeichnet, dass** die Matte eine Webmatte ist, in der die Kapillarmembranen und die Verbindungsfäden miteinander verwoben sind.

8. Wundversorgungssystem nach einem oder mehreren der Ansprüche 4 bis 6, **dadurch gekennzeichnet, dass** die Matte eine Wirkmatte ist, in der die Kapillarmembranen und die Verbindungsfäden miteinander verwirkt sind.

9. Wundversorgungssystem nach einem oder mehreren der Ansprüche 1 bis 8, **dadurch gekennzeichnet, dass** die mindestens eine gemeinsame Versorgungsleitung ein flexibler Silikonschlauch ist.

10. Wundversorgungssystem nach einem oder mehreren der Ansprüche 1 bis 9, **dadurch gekennzeichnet, dass** es mindestens eine weitere Anordnung von Kapillarmembranen umfasst.

11. Wundversorgungssystem nach einem oder mehreren der Ansprüche 1 bis 10, **dadurch gekennzeichnet, dass** es ein Drainagesystem umfasst.

12. Wundversorgungssystem nach einem oder mehreren der Ansprüche 1 bis 11, **dadurch gekennzeichnet, dass** es des Weiteren eine taschenförmige Wundauflage umfasst, wobei die taschenförmige Wundauflage an ihrem äußeren Rand geschlossen ist und eine Oberseite, eine Unterseite und ein Tascheninneres aufweist, wobei die Unterseite und die Oberseite jeweils aus einem flächigen Material ausgebildet sind und die Unterseite permeabel für Fluide ist, wobei die Anordnung aus parallel zueinander angeordneten Kapillarmembranen im Tascheninneren angeordnet ist und wobei die mindestens eine Versorgungsleitung außerhalb der taschenförmigen Wundauflage mit einer Einheit zur Versorgung bzw. Entsorgung verbindbar ist.

13. Wundversorgungssystem nach Anspruch 12, **dadurch gekennzeichnet, dass** es ein Drainagesystem umfasst, welches im Tascheninneren angeordnet und welches zur Entfernung von Exsudat aus zu behandelnden Wunden geeignet ist.

14. Wundversorgungssystem nach Anspruch 13, **dadurch gekennzeichnet, dass** das Drainagesystem ein Drainagekatheter ist, der über eine an seinen Querschnitt fluiddicht angepasste Durchtrittsöffnung aus der taschenförmigen Wundauflage herausführt und mit einer Unterdruckeinheit verbindbar ist.

**15.** Wundversorgungssystem nach einem oder mehreren der Ansprüche 12 bis 14, **dadurch gekennzeichnet, dass** die Unterseite der taschenförmigen Wundauflage aus einem vliesförmigen, flächigen Material oder einer semipermeablen, mikroporösen Flachmembran ausgebildet ist.

**Claims**

**1.** Wound treatment system, comprising a single-layer arrangement of capillary membranes (3) arranged parallel to one another, having a porous semipermeable wall, and a lumen, and at least one open end,

> - wherein the capillary membranes have an external diameter in a range of from 50 to 500 $\mu$m, and a wall thickness in a range of from 5 to 1000 $\mu$m,
> - wherein the capillary membranes are connected by their at least one open end to at least one common supply line having a wall and a lumen,

**characterized in that**

> - the capillary membranes, with their at least one open ends, are embedded fluid-tight at their outer periphery in the wall of the at least one common supply line (5, 6) so that a fluid connection exists between the lumen of the supply line and the lumen of the capillary membranes,
> - the arrangement of capillary membranes arranged parallel to one another furthermore comprises a plurality of interspaced connecting elements (4) which run parallel to one another, by means of which the capillary membranes are connected to one another to form a mat and are held at a distance from one another by the connecting elements,
> - the spacing of the capillary membranes from one another in the mat is 1 to 10 times the external diameter of the capillary membranes, wherein the distance is measured from the longitudinal axes of the capillary membranes, and
> - the distance between the connecting elements is in a range of from 1 to 50 mm.

**2.** Wound treatment system according to claim 1, **characterized in that** the capillary membrane arrangement is connected to two supply lines, wherein the capillary membranes are embedded with their opposite ends into a respective supply line.

**3.** Wound treatment system according to claim 1 or 2, **characterized in that** the capillary membranes have a transmembrane flow for water in a range of from 0.01 to 50 ml/(min·cm$^2$·bar).

**4.** Wound treatment system according to one or more of claims 1 to 3,
**characterized in that** the capillary membranes are connected by means of yarn-like connecting elements to form a mat.

**5.** Wound treatment system according to claim 4, **characterized in that** the yarn-like connecting elements are multifilament textile yarns.

**6.** Wound treatment system according to claim 5, **characterized in that** the multifilament textile yarns are multifilament polyester yarns, polypropylene yarns, or polytetrafluoroethylene yarns.

**7.** Wound treatment system according to one or more of claims 4 to 6,
**characterized in that** the mat is a woven mat in which the capillary membranes and the connecting threads are interwoven.

**8.** Wound treatment system according to one or more of claims 4 to 6,
**characterized in that** the mat is a knit mat in which the capillary membranes and the connecting threads are knitted together with one another.

**9.** Wound treatment system according to one or more of claims 1 to 8
**characterized in that** the at least one common supply line is a flexible silicone hose.

**10.** Wound treatment system according to one or more of claims 1 to 9,

**characterized in that** it comprises at least one further arrangement of capillary membranes.

11. Wound treatment system according to one or more of claims 1 to 10,
**characterized in that** it comprises a drainage system.

12. Wound treatment system according to one or more of claims 1 to 11,
**characterized in that** it furthermore comprises a pocket-like wound dressing, wherein the pocket-shaped wound dressing is closed at its outer edge and has a top side, a bottom side, and a pocket interior, wherein the bottom side and the top side are respectively formed from a flat material, and the underside is permeable to fluids, wherein the arrangement of capillary membranes arranged parallel to one another is arranged in the pocket interior, and wherein the at least one supply line can be connected outside of the pocket-like wound dressing to a unit for supply or disposal.

13. Wound treatment system according to claim 12, **characterized in that** it comprises a drainage system which is arranged in the pocket interior and which is suitable for removing exudate from wounds to be treated.

14. Wound treatment system according to claim 13, **characterized in that** the drainage system is a drainage catheter which exits the pocket-like wound dressing via a passage opening that is adapted to be sealed against fluids at its cross-section, and which can be connected to a negative pressure unit.

15. Wound treatment system according to one or more of claims 12 to 14,
**characterized in that** the bottom side of the pocket-like wound dressing is formed from a non-woven, flat material or a semipermeable, microporous flat membrane.

**Revendications**

1. Système de traitement des plaies, comprenant un agencement monocouche de membranes capillaires parallèles les unes aux autres (3) avec une paroi poreuse, semi-perméable et une lumière et au moins une extrémité ouverte,

   - dans lequel les membranes capillaires présentent un diamètre extérieur dans la plage de 50 à 500 $\mu$m et une épaisseur de paroi dans la plage de 5 à 1 000 $\mu$m,
   - dans lequel les membranes capillaires sont reliées par leur au moins une extrémité ouverte avec au moins une tubulure d'alimentation commune avec une paroi et une lumière,

   **caractérisé en ce que**,

   - les membranes capillaires sont intégrées avec leur au moins une extrémité ouverte à leur pourtour externe de manière étanche aux fluides dans la paroi de l'au moins une tubulure d'alimentation commune (5, 6), de sorte qu'une liaison fluidique existe entre la lumière de la tubulure d'alimentation et la lumière des membranes capillaires,
   - **en ce que** l'agencement de membranes capillaires disposées parallèlement les unes aux autres présente en outre plusieurs éléments de liaison (4) espacés les uns des autres et s'étendant parallèlement les uns aux autres, au moyen desquels les membranes capillaires sont liées les unes aux autres en un tissu et sont maintenues à distance les unes des autres par les éléments de liaison,
   - **en ce que** la distance des membranes capillaires les unes par rapport aux autres dans le tissu représente 1 à 10 fois le diamètre extérieur des membranes capillaires, la distance des axes longitudinaux des membranes capillaires étant mesurée et
   - **en ce que** la distance des éléments de liaison les uns par rapport aux autres est dans une plage de 1 à 50 mm.

2. Système de traitement des plaies selon la revendication 1, **caractérisé en ce que** l'agencement de membranes capillaires est relié avec deux tubulures d'alimentation, les membranes capillaires étant intégrées avec leurs extrémités opposées respectivement dans une tubulure d'alimentation.

3. Système de traitement des plaies selon la revendication 1 ou 2,
**caractérisé en ce que** les membranes capillaires présentent un flux transmembranaire pour l'eau dans la plage de 0,01 à 50 ml/(min•cm$^2$•bar).

4. Système de traitement des plaies selon une ou plusieurs des revendications 1 à 3, **caractérisé en ce que** les membranes capillaires sont liées en un tissu par des éléments de liaison en forme de fils.

5. Système de traitement des plaies selon la revendication 4, **caractérisé en ce que** les éléments de liaison en forme de fil sont des fils textiles à plusieurs filaments.

6. Système de traitement des plaies selon la revendication 5, **caractérisé en ce que** les fils textiles à plusieurs filaments sont des fils de polyester, des fils de polypropylène ou des fils de polytétrafluoréthylène multifils.

7. Système de traitement des plaies selon une ou plusieurs des revendications 4 à 6, **caractérisé en ce que** le tissu est un tissu tissé, dans lequel les membranes capillaires et les fils de liaison sont tissés les uns avec les autres.

8. Système de traitement des plaies selon une ou plusieurs des revendications 4 à 6, **caractérisé en ce que** le tissu est un tissu actif, dans lequel les membranes capillaires et les fils de liaison sont tricotés les uns avec les autres.

9. Système de traitement des plaies selon une ou plusieurs des revendications 1 à 8, **caractérisé en ce que** ladite au moins une tubulure d'alimentation commune est un tube flexible en silicone.

10. Système de traitement des plaies selon une ou plusieurs des revendications 1 à 9, **caractérisé en ce qu'**il contient au moins un agencement supplémentaire de membranes capillaires.

11. Système de traitement des plaies selon une ou plusieurs des revendications 1 à 10, **caractérisé en ce qu'**il contient un système de drainage.

12. Système de traitement des plaies selon une ou plusieurs des revendications 1 à 11, **caractérisé en ce qu'**il comporte en outre un pansement en forme de poche, le pansement en forme de poche étant fermé à son bord extérieur et présentant une face supérieure, une face inférieure et un intérieur de poche, dans lequel la face inférieure et la face supérieure sont constituées chacune d'un matériau plat et la face inférieure est perméable aux fluides et dans lequel l'agencement de membranes capillaires parallèles les unes aux autres est disposé à l'intérieur de la poche et dans lequel l'au moins une tubulure d'alimentation à l'extérieur du pansement en forme de poche peut être reliée à une unité d'alimentation ou d'évacuation.

13. Système de traitement des plaies selon la revendication 12, **caractérisé en ce qu'**il comporte un système de drainage, qui est disposé à l'intérieur de la poche et qui convient pour l'élimination de l'exsudant des plaies à traiter.

14. Système de traitement des plaies selon la revendication 13, **caractérisé en ce que** le système de drainage est un cathéter de drainage, qui fait saillie par le biais d'un orifice de passage adapté à sa section transversale de manière étanche aux fluides hors du pansement en forme de poche et qui peut être relié à une unité de dépression.

15. Système de traitement des plaies selon une ou plusieurs des revendications 12 à 14, **caractérisé en ce que** la face inférieure du pansement en forme de poche est constituée d'un matériau plat en feuillet non tissé ou d'une membrane plane microporeuse semi-perméable.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006042732
- DE 3839567
- DE 4308850
- EP 0442147 A
- EP 1144096 A
- EP 0299381 A
- DE 2833493 A